# EUROPÄISCHE PATENTSCHRIFT

(11) **EP 3 383 317 B1**
(45) Veröffentlichungstag und Bekanntmachung des Hinweises auf die Patenterteilung: **15.09.2021**
(21) Anmeldenummer: 16801777.0
(22) Anmeldetag: 28.11.2016
(51) Int. Cl.: A61F 2/16

(54) **INTRAOKULARLINSEN-KASSETTE MIT GLEITMITTEL-ZUFÜHRKANAL UND INJEKTOR MIT KASSETTE**
INTRAOCULAR LENS CARTRIDGE COMPRISING A LUBRICANT FEED DUCT, AND INJECTOR COMPRISING A CARTRIDGE
CASSETTE POUR LENTILLE INTRAOCULAIRE, POURVUE D'UN CANAL D'ALIMENTATION EN LUBRIFIANT, ET INJECTEUR ÉQUIPÉ D'UNE TELLE CASSETTE

(30) Priorität: 03.12.2015 DE 102015224141
(43) Veröffentlichungstag der Anmeldung: 10.10.2018
(73) Patentinhaber: Carl Zeiss Meditec AG, 07745 Jena (DE)
(72) Erfinder: MÜLLER, Marco, 10437 Berlin (DE)
(74) Vertreter: Carl Zeiss AG - Patentabteilung
(86) Internationale Anmeldenummer: PCT/EP2016/078944
(87) Internationale Veröffentlichungsnummer: WO 2017/093161

(56) Entgegenhaltungen:
- WO-A1-2013/038689
- WO-A1-2013/168410
- WO-A1-2014/208507
- WO-A2-2015/070358
- US-A1- 2010 130 985

## Beschreibung

Die Erfindung betrifft eine Kassette zur Aufnahme einer Intraokularlinse für einen Injektor sowie einen Injektor mit einer solchen Kassette zum Einführen der Intraokularlinse in ein Auge.

Es ist bekannt, dass viskoelastische Materialien beziehungsweise Fluide, die auch als OVD (Ophthalmo-viscosurgical devices) bezeichnet werden, in der operativen Augenheilkunde umfänglich Anwendung finden. Bei Kataraktoperationen, bei denen eine Augenlinse zertrümmert und abgesaugt wird und dann eine Intraokularlinse eingesetzt wird, werden diese Materialien verwendet. Sie werden dabei beim operativen Eingriff auch in das Auge injiziert. Dadurch wird beispielsweise unter anderem die Stabilität der vorderen Augenkammer aufrecht erhalten. Darüber hinaus wird durch diese Materialien das Gewebe der Intraokularlinse geschützt und das Endothel der Hornhaut beziehungsweise der Kornea ebenfalls geschützt. Darüber hinaus wird dadurch in diesem operativen Eingriff an dem Auge Volumen bereitgestellt, um Gewebe präzise und zuverlässig entnehmen zu können.

Diese Operations-Hilfsfluide sind jedoch nach dem Ende des operativen Eingriffs wieder vollständig aus dem Auge zu entfernen, denn bei einem Verbleib im Auge können Komplikationen auftreten und sich insbesondere auch der Augendruck unerwünscht erhöhen.

Es ist aus der US 8 123 804 B2 eine Kassette für eine Intraokularlinse bekannt, die einen an einem wannenartigen Teil der Kassette schwenkbar angeordneten, plattenartigen Deckel aufweist, der den Volumenbereich des wannenartigen Teils verschließt. Durch den Deckel und das wannenartige Teil ist die Kassette gebildet. In dem Volumenbereich ist die Intraokularlinse aufgenommen, bevor sie ausgeschoben wird und mit einem Injektor in das Auge implantiert wird. Dieser Deckel weist ein Loch auf, welches nach außen führt und durch welches im geschlossenen Zustand der Kassette ein Gleitmittel in den Volumenbereich eingebracht werden kann.

Ein wesentlicher Nachteil ist darin zu sehen, dass durch das kleine und somit in Lochachse wenig tiefe und geradlinige Loch beim Einfüllen des Gleitmittels auch die Intraokularlinse beschädigt werden kann. Das Einführen erfolgt üblicherweise mit einer Spritze mit einer Nadel, die in das Loch eingeführt wird. Dabei kann leicht die Nadel zu weit eingeführt werden und die Intraokularlinse berühren, so dass sie verkratzt und/oder verrutscht werden kann. Das Verrutschen führt dann auch dazu, dass das dortige nachfolgende Falten der Intraokularlinse in der Injektorspitze beim Ausschiebevorgang nicht mehr so erfolgt wie es sein sollte, da die Ausgangslage vor dem Falten verändert wurde. Dies kann zu Problemen bei der Einführung ins Auge führen. Darüber hinaus ist bei diesem Stand der Technik die Kassette mit ihrem Deckel und wannenartigen Teil derart ausgebildet, dass ein Falten der Intraokularlinse erst in der an die Kassette anschließende Injektorspitze ermöglicht ist, so dass durch d as Schließen des plattenartigen Deckels der Kassette keinerlei Einfluss auf die Lage und Form der Intraokularlinse in dem Volumenbereich erfolgt.

Darüber hinaus ist aus der EP 1 808 150 A1 eine Kassette für eine Intraokularlinse bekannt, welche zwei relativ zueinander schwenkbare Teile aufweist, die im geschlossenen Zustand einen Aufnahmebereich für die Intraokularlinse bilden. An einem hinteren Bereich der Teile und somit benachbart zu einem die Teile schwenkbar verbindenden Scharnier ist ein Loch zum Einfüllen eines Gleitmittels ausgebildet. Bei dieser Ausführung tritt das Problem auf, dass das Einfüllen des Gleitmittels das Falten der Linse behindert. Dadurch wird die gewünschte Faltrichtung der Linse hin zum Scharnier wesentlich beeinträchtigt und gehemmt, so dass die gefaltete Endstellung der Intraokularlinse in der Kassette nicht oder nur unzureichend erreicht wird, was wiederum nachteilig für den weiteren Ausschiebevorgang ist und zu Beschädigungen der Linse oder einer unerwünschten Ausschiebelage am vorderen Ende der Injektorspitze führt.

Aus der WO 2015/070358 A2 ist eine Vorrichtung zur Aufnahme einer Intraokularlinse bekannt. Dort ist eine Kartusche gezeigt, welche zwei direkt miteinander schwenkbar verbundene Halbschalen aufweist, an denen jeweils noch ein Flügel ausgebildet ist. Zwischen den Halbschalen erstreckt sich ein Deckel, der eine Aussparung aufweist, durch welche ein Gleitmittel in eine Kammer, in welcher die Intraokularlinse angeordnet ist, zuführbar ist. Die Zuführung kann jedoch nur im geöffneten Zustand der Flügel und somit auch der Halbschalen erfolgen. Dadurch kann der nur einseitig an nur einer Halbschale mit einem Filmscharnier befestigte Deckel angehoben werden und das Gleitmittel aus der Kammer unerwünscht entweichen. Des Weiteren ist die Aussparung in dem Deckel schwer zu treffen, so dass die Nadel einer Gleitmittel-Zuführungseinheit auch abrutschen kann. Darüber hinaus ist die Aussparung geometrisch nachteilig, da die Nadel der Gleitmittel-Zuführeinheit leicht hindurch geführt werden kann und dann die Intraokularlinse beschädigen kann.

In der WO 2003/044946 A2 ist eine Vorrichtung zum Falten einer Intraokularlinse sowie ein Aufbewahrungssystem für eine Intraokularlinse beschrieben.

Es ist eine Aufgabe der vorliegenden Erfindung, eine Kassette für eine Intraokularlinse zu schaffen, die im geschlossenen Zustand der Kassette eine sichere externe Zuführung des Gleitmittels ermöglicht und die Gefahr einer Beschädigung der Intraokularlinse reduziert. Es ist ferner eine Aufgabe, einen Injektor mit einer solchen Kassette zu schaffen.

Eine erfindungsgemäße Kassette ist zur Aufnahme einer Intraokularlinse ausgebildet. Sie ist daher eine Intraokularlinsen-Aufnahmekassette. Die Kassette ist für einen Injektor zum Einführen der Intraokularlinse in ein Auge ausgebildet. Die Kassette weist zwei um eine Längsachse der Kassette relativ zueinander schwenkbare Kassettenteile auf. Ein Kassettenteil weist dabei ein rinnenartiges Basiselement und einen daran anschließenden plattenartigen Flügel auf. Ein weiteres Kassettenteil weist ebenfalls ein rinnenartiges Basiselement und einen daran anschließenden plattenartigen Flügel auf. Die Kassettenteile sind jeweils insbesondere einstückig ausgebildet. Durch die Basiselemente wird im geschlossenen Zustand der Kassette mindestens ein Teil einer insbesondere röhrenförmigen Aufnahmekammer für die Intraokularlinse und somit eine Intraokularlinsen-Aufnahmekammer gebildet. Die Basiselemente sind bezüglich ihrer rinnenartigen Ausgestaltung somit als bauchige Schalenteile gebildet, die somit insbesondere zueinander entgegengesetzt gekrümmte und eventuell gebogene Elemente sind. Die Kassette weist darüber hinaus eine Gleitmittel-Zuführeinrichtung auf, mit welcher für die Intraokularlinse bei geschlossener Kassette und dann auch von außerhalb der Kassette ein fluides Gleitmittel vorzugsweise radial in die Aufnahmekammer zuführbar ist.

Ein wesentlicher Gedanke der Erfindung ist darin zu sehen, dass die Gleitmittel-Zuführeinrichtung einen ersten Zuführkanal aufweist, der in zumindest einem Kassettenteil ausgebildet ist und in die Aufnahmekammer mündet. Durch eine derartige Ausgestaltung der Kassette kann auch im geschlossenen Zustand dieser Kassette und somit bei zusammengefügten oder maximal zueinander verschwenkten beziehungsweise aufeinander zu bewegten Kassettenteilen dennoch ein radiales Einbringen von Gleitmittel durch eines der Kassettenteile hindurch erfolgen. Es muss daher nicht mehr in Richtung der Längsachse der Kassette betrachtet von einem frontseitig offenen Ende oder einem rückseitig offenen Ende Gleitmittel zugeführt werden. Stattdessen ist es mit der erfindungsgemäßen Kassette nun möglich, Gleitmittel von außerhalb der Kassette in einem von 0° und von 180° abweichenden Winkel zur Längsachse der Kassette zuzuführen. Diese Zuführung des Gleitmittels von außerhalb der Kassette erfolgt somit innerhalb eines Längenabschnitts entlang der Längsachse betrachtet, welcher von einem vorderen und einem hinteren Ende der Kassette in dieser Längsrichtung betrachtet beabstandet ist. Durch eine derartige Ausgestaltung kann eine gleichmäßigere Zuführung des Gleitmittels in die Aufnahmekammer erfolgen, sodass sich darin auch eine bessere Verteilung des Gleitmittels ergibt. Diese gleichmäßigere Verteilung und auch gleichmäßigere Einführung des Gleitmittels wird im besonderen Maße durch diesen ersten Zuführkanal erreicht. Darüber hinaus ermöglicht dieser erste Zuführkanal mit seiner diesbezüglich definierten Länge auch, dass ein Hilfswerkzeug zum Einbringen des Gleitmittels in den ersten Zuführkanal auch nicht bis zur Aufnahmekammer gelangt. Dadurch wird zusätzlich erreicht, dass eine Beschädigung einer in der Kassette enthaltenen Intraokularlinse, beispielsweise durch eine Injektionsnadel dieses Hilfswerkzeugs, verhindert wird, da die Injektionsnadel nicht mehr bis zur Intraokularlinse gelangen und berühren kann. Somit kann es auch nicht dazu kommen, dass die Intraokularlinse in der Aufnahmekammer ungewollt verschoben wird, wie dies bei bisher üblichen Kassetten noch möglich ist. Mit der erfindungsgemäßen Kassette lässt sich daher vermeiden, dass die Intraokularlinse nur durch das Zuführen von Gleitmittel teilweise vorgefaltet wird.

Gemäß der Erfindung ist vorgesehen, dass der erste Zuführkanal in einem sich in die Aufnahmekammer hinein erstreckenden Steg eines Kassettenteils ausgebildet ist. Dadurch wird eine sehr exponierte Komponente, nämlich der Steg, bereitgestellt, der ähnlich einem Podest ausgebildet ist. Dadurch ist es möglich, das Gleitmittel gezielt an einen Bereich innerhalb der Aufnahmekammer zu befördern. Damit kann sich das Gleitmittel besser von einer Position im Inneren der Aufnahmekammer zum Rand der Aufnahmekammer hin verteilen. Dies reduziert die Gefahr deutlich, dass Bereiche in der Aufnahmekammer vom Gleitmittel nicht erreicht werden, woraufhin die zu befördernde Intraokularlinse beim anschließenden Transport im Injektorrohr verkratzen könnte.

In vorteilhafter Weise ist vorgesehen, dass der erste Zuführkanal geradlinig ausgebildet ist. Dadurch wird erreicht, dass sich das Gleitmittel gleichmäßig in die Aufnahmekammer der Kassette einführen lässt. Es kommt somit zu keiner ruckartigen oder pulsartigen Zufuhr des Gleitmittels. Zudem ist bei einem geradlinigen Zuführkanal die Wahrscheinlichkeit gering, dass das Gleitmittel den Zuführkanal verstopft.

Vorzugsweise ist vorgesehen, dass sich der Steg parallel zur Längsachse erstreckt und in Richtung der Längsachse betrachtet sich zumindest über die Hälfte der Länge der Aufnahmekammer erstreckt. Insbesondere erstreckt sich dieser Steg über zumindest 80 % der Länge der Aufnahmekammer. Der Steg erstreckt sich insbesondere vollständig geradlinig. Durch eine derartige geometrische Ausgestaltung des Stegs als Schiene wird die Stabilität des Kassettenteils erhöht, insbesondere an einem Übergang zwischen dem Basiselement und dem Flügel eines Kassettenteils.

Es ist insbesondere vorgesehen, dass der an einem Kassettenteil ausgebildete Steg eine dem anderen Kassettenteil zugewandte Oberseite beziehungsweise Innenseite aufweist, in welcher der erste Zuführkanal zur Oberseite hin offen und bevorzugt rinnenartig ausgebildet ist.

Grundsätzlich ist mit einem Zuführkanal ein in Umlaufrichtung um die Längserstreckung des Zuführkanals nicht geschlossener Kanal zu verstehen.

Bei dieser Ausführungsform kann ein anderer Steg in dem anderen Kassettenteil genutzt werden, um den rinnenartigen Zuführkanal zuverlässig abzudecken. Ein unerwünschtes Ausquellen des in den ersten Zuführkanal eingebrachten Gleitmittels kann dadurch verhindert werden. Darüber hinaus ist es durch diese Ausgestaltung möglich, einen einfachen Zugang zu diesem Zuführkanal zu erhalten und diesen einzusehen beziehungsweise darin Handhabungen vorzunehmen.

In vorteilhafter Weise ist diese Oberseite dann bündig in eine Innenseite des daran anschließenden Flügels übergehend ausgebildet. Dadurch lässt sich eine besonders großflächige mechanische Kontaktierung mit dem anderen Kassettenteil erreichen, sodass ein Verteilen oder unerwünschtes Ausquellen des Gleitmittels aus dem Zuführkanal außerhalb der Aufnahmekammer verhindert ist.

Vorzugsweise ist vorgesehen, dass der Steg keilartig ausgebildet ist. Diese spezifische Geometrie ermöglicht einerseits eine großflächige und mechanisch stabile Anlage des anderen Kassettenteils, insbesondere eines an dem anderen Kassettenteil angebrachten Stegs, sodass die geschlossene Stellung der Kassette auch besonders stabil gehalten ist. Darüber hinaus ist durch diese Keilform auch erreicht, dass eine dem anderen Kassettenteil und somit insbesondere dem anderen Steg am anderen Kassettenteil abgewandte Flanke schräg gestellt ist. Eine solche Geometrie kann für den mechanischen Kontakt mit der Intraokularlinse genutzt werden, sodass beim Schließen der Kassette das Vorfalten der Intraokularlinse in der Aufnahmekammer in einer vorbestimmten Weise erfolgt.

Insbesondere ist vorgesehen, dass an einem innenseitigen Übergang zwischen einem Basiselement und einem Flügel eines Kassettenteils dieser Steg ausgebildet ist. Diese exponierte Lage des Stegs begünstigt den Faltvorgang der Intraokularlinse in der Kassette, wenn diese geschlossen wird.

Der Steg ist insbesondere auch ein Faltanschlagelement, durch welches der Faltvorgang in azimutaler Richtung um die Längsachse der Kassette unterstützt wird. Dies ist derart zu verstehen, dass ein zu starker und somit unerwünschter Faltungszustand der Intraokularlinse in der Aufnahmekammer vermieden wird. In azimutaler Richtung begrenzt somit dieser Steg das weitere Falten beziehungsweise Aufrollen der Intraokularlinse. Werden die Kassettenteile aufeinander zu bewegt, sodass die Kassette geschlossen wird, erfolgt ein Vorfalten der Intraokularlinse in der Aufnahmekammer, wobei der Steg an einem Kassettenteil, vorzugsweise jeweils einen Steg an jeweils einem Kassettenteil, ein azimutales Faltanschlagelement bildet.

Bei einer Betrachtung in einer Ebene senkrecht zur Längsachse der Kassette weist ein Steg somit eine Dreiecksform als bevorzugte Geometrie auf.

Insbesondere ist vorgesehen, dass der erste Zuführkanal im geschlossenen Zustand der Kassette von einer ebenen Oberseite beziehungsweise Innenseite eines an dem anderen Kassettenteil ausgebildeten weiteren Stegs abgedeckt ist. Dies ist vorteilhaft, da der erste Zuführkanal somit bei geschlossener Kassette zwangsläufig auch geschlossen ist und dann bei einem nach dem Erreichen des geschlossenen Zustands erfolgenden Zuführen von Gleitmittel über den Zuführkanal kein radialer Austritt des Gleitmittels erfolgen kann. Im geschlossenen Zustand der Kassette ist somit der Zuführkanal in radialer Richtung geschlossen. Wenn der Zuführkanal rinnenartig ausgebildet ist, ist dieser im geöffneten Zustand der Kassette durch die Rinnenform in radialer Richtung zur Längsachse des Zuführkanals frei zugänglich.

Vorzugsweise ist vorgesehen, dass in dem anderen Kassettenteil ein sich in den Aufnahmeraum beziehungsweise die Aufnahmekammer erstreckender weiterer Steg ausgebildet ist, in dem ein zweiter Zuführkanal ausgebildet ist. Im geschlossenen Zustand der Kassette sind die Zuführkanäle deckungsgleich angeordnet und decken sich gegenseitig somit auch ab. Das Volumen eines aus den beiden Zuführkanälen gebildeten Gesamtzuführkanals ist dann gegenüber einem einzigen Zuführkanal vergrößert. Dadurch kann eine größere Menge an Gleitmittel schneller in die Aufnahmekammer eingebracht werden. Dennoch ist im geöffneten Zustand der Kassette jeder der Zuführkanäle frei zugänglich, wenn diese rinnenartig ausgebildet sind.

In einer vorteilhaften Ausführung ist vorgesehen, dass der erste Zuführkanal in einem Kassettenteil ausgebildet ist und ein durchgängiges Einfüllloch an dem Kassettenteil oder an dem weiteren Kassettenteil ausgebildet ist. Das Einfüllloch ist im geschlossenen Zustand der Kassette an dem ersten Zuführkanal mündend ausgebildet und im geschlossenen Zustand der Kassette mündet dieses Einfüllloch nach außen und ist somit von außen zugänglich. Dieses Einfüllloch erlaubt somit das Einfüllen von Gleitmittel in die Kassette. Andererseits ist der erste Zuführkanal bei geschlossener Kassette von außen nicht direkt zugänglich beziehungsweise mündet nicht direkt nach außen. Erst durch die Kopplung und somit das Zusammenwirken zwischen dem Einfüllloch und dem ersten Zuführkanal wird ein Gesamtweg erzeugt, durch den das Gleitmittel von außerhalb von der Kassette in die Aufnahmekammer leitbar ist. Durch diese besonders vorteilhafte Ausführung kann in unterschiedlichen Kassettenteilen jeweils eine Teilkomponente der Gleitmittel-Zuführeinrichtung geschaffen werden, wobei jeweils die bereits oben genannten Vorteile für sich generiert werden. Durch diese Ausgestaltung ist im besonderen Maße die Zuführung des Gleitmittels in die Aufnahmekammer ohne unerwünschte Kontaktierung und ohne unerwünschte Verschiebung der Intraokularlinse durch ein Hilfswerkzeug zur Einführung des Gleitmittels erreicht.

Es kann vorgesehen sein, dass im geschlossenen Zustand der Kassette eine Lochachse des Einfülllochs und eine Längsachse des ersten Zuführkanals nicht parallel zueinander und nicht koaxial zueinander sind. Dies ist eine weitere, sehr vorteilhafte Ausführung, denn durch diese Anordnung in einem Winkel von unterschiedlich 0° und unterschiedlich 180° ist auf dem Gesamtweg, auf dem das Gleitmittel von außerhalb von der Kassette in die Aufnahmekammer gebracht wird, zumindest ein Knick ausgebildet. Durch diese Ausgestaltung ist es dann erreicht, dass beispielsweise eine Injektionsnadel eines Hilfswerkzeugs niemals bis zur Aufnahmekammer gelangen und dort die Intraokularlinse berühren könnte. Maximal kann diese Injektionsnadel dann eine Einstechtiefe erreichen, die der Tiefe des Einfülllochs entspricht, wodurch dann aufgrund des anderweitigen Verlaufs beziehungsweise der anderweitigen Orientierung des zumindest ersten Zuführkanals ein Weiterbewegen dieser Injektionsnadel verhindert ist.

In vorteilhafter Weise ist vorgesehen, dass die Lochachse des Einfülllochs zu einer Längsachse des ersten Zuführkanals in einem Winkel zwischen 90° und 160°, insbesondere zwischen 100° und 130°, geneigt angeordnet ist.

In vorteilhafter Weise ist vorgesehen, dass eine Lochachse des Einfülllochs in einem Winkel zwischen 90° und 160° zu einer Längsachse des Zuführkanals desjenigen Kassettenteils geneigt angeordnet ist, in dem das Einfüllloch ausgebildet ist. Dies ermöglicht ein einfaches Positionieren eines Hilfswerkzeugs, insbesondere einer Injektionsnadel eines Hilfswerkzeugs, zur Kassette und begünstigt auch das Leiten des Gleitmittels von dem Einfüllloch in einen Zuführkanal und von dort in die Aufnahmekammer.

Es kann vorgesehen sein, dass zumindest der erste Zuführkanal in einem Winkel zwischen 60° und 120°, insbesondere zwischen 80° und 100°, zu der Längsachse der Kassette orientiert ist. Dadurch wird ein relativ kurzer erster Zuführkanal gebildet. Ein Verstopfen des Zuführkanals mit dem Gleitmittel ist dadurch vermieden.

Bevorzugt ist der erste Zuführkanal entlang seiner Kanalachse nichtgeradlinig ausgebildet und zumindest einmal gekrümmt oder in einem Winkelversatz verlaufend ausgebildet. Eine derartige Ausgestaltung erhöht den Strömungswiderstand des Zuführkanals und kann bei einem Gleitmittel mit geringer Viskosität die Geschwindigkeit begrenzen, mit der Gleitmittel in die Aufnahmekammer eindringt.

In vorteilhafter Weise ist vorgesehen, dass der zumindest erste Zuführkanal mit einem der Aufnahmekammer abgewandten Ende im geschlossenen Zustand der Kassette an dem Flügel eines Kassettenteils nach außen hin mündet und der erste Zuführkanal insbesondere vollständig geradlinig ausgebildet ist. Bei einer derartigen Ausführung ist dann ein Eingang des zumindest ersten Zuführkanals zwischen den beiden aneinander anliegenden Flügeln der Kassettenteile ausgebildet. In vorteilhafter Weise ist dann hier vorgesehen, dass die den Basiselementen abgewandten Enden der Flügel leicht voneinander weg gekrümmt sind und somit nicht direkt kontaktiert sind. Dadurch wird eine schnabelartige beziehungsweise Y-förmige Aufspreizung an diesen den Basiselementen abgewandten Enden der Kassettenteile erreicht, wodurch ein Hilfswerkzeug leicht zu diesem Eingang dieses Zuführkanals zugeführt werden kann.

Es kann auch vorgesehen sein, dass sich zumindest der erste Zuführkanal teilweise parallel zur Längsachse der Kassette erstreckt. Dadurch kann das Gleitmittel in Abhängigkeit von der Geometrie der Intraokularlinse optimal in die Aufnahmekammer zugeführt werden.

In einer vorteilhaften Ausführung ist vorgesehen, dass ein der Aufnahmekammer zugewandtes Ende des zumindest ersten Zuführkanals verjüngt ausgebildet ist. Dadurch kann in noch verbesserter Weise ein Eindringen des Hilfswerkzeugs, insbesondere einer Injektionsnadel eines Hilfswerkzeugs, in die Aufnahmekammer vermieden werden. Es ist somit diese Verjüngung insbesondere derart ausgebildet, dass ein Innendurchmesser kleiner ist als ein Außendurchmesser einer derartigen Injektionsnadel eines Hilfswerkzeugs.

Besonders vorteilhaft ist diese Ausführung dann, wenn der zumindest erste Zuführkanal vollständig geradlinig ausgebildet ist.

Vorzugsweise ist die Kassette mit den Kassettenteilen einstückig ausgebildet, insbesondere aus Kunststoff. Die Kassettenteile sind vorzugsweise direkt miteinander verbunden. Es kann dazu vorgesehen sein, dass dazu mindestens ein Filmscharnier ausgebildet ist. Dieses Filmscharnier ist insbesondere an Enden der Basiselemente ausgebildet, die den jeweiligen Flügeln der Kassettenteile abgewandt sind.

In einer weiteren vorteilhaften Ausführung kann vorgesehen sein, dass die radiale Erstreckung und somit eine Erstreckung senkrecht zu einer Längsachse der Kassette eines Stegs an einem Kassettenteil unterschiedlich zu dieser radialen Erstreckung des Stegs an dem anderen Kassettenteil ist. Es wird somit an den der Aufnahmekammer zugewandten Enden beziehungsweise Fronträndern dieser Stege ein radialer Versatz bzw. Überstand ausgebildet, wodurch eine alternative Ausgestaltung bezüglich des Faltanschlagelements erreicht ist. Auch kann sich dann ein Zuführkanal an derjenigen Oberseite eines Stegs ausbilden, der sich radial weiter in die Aufnahmekammer hineinerstreckt, sodass dieser Zuführkanal abschnittsweise dann auch radial freiliegend in die Aufnahmekammer mündet, da er sich auch in dem Versatz bzw. dem Überstand erstreckt und insbesondere dann über den Versatz in die Aufnahmekammer mündet. Eine umfänglichere Gleitmitteleinführung ist dadurch ermöglicht.

Bevorzugt erstreckt sich der erste Zuführkanal entlang einer gesamten Länge eines sich in die Aufnahmekammer hinein erstreckenden Steges eines Kassettenteiles. Damit kann ein über eine relativ große Strecke gleichmäßiger Eintrag von Gleitmittel in den Aufnahmeraum der Kassette erreicht werden.

Des Weiteren betrifft die Erfindung auch einen Injektor zum Einführen einer Intraokularlinse in ein Auge, welcher eine Kassette gemäß der oben genannten Erfindung oder einer vorteilhaften Ausgestaltung davon aufweist. Die Kassette kann als separates Teil bereitgestellt werden, welches dann zum Einsatz in einem Ladebereich des Injektors, insbesondere in einem Ladebereich einer Injektorröhre, ausgebildet ist.

In vorteilhafter Weise ist vorgesehen, dass die Kassette integral und somit einstückig mit einer Injektorspitze des Injektors zum Implantieren einer Intraokularlinse in ein Auge ausgebildet ist. Die Injektorspitze stellt dabei das Bauteil des Injektors dar, welches mit dem vorderen Ende in das Auge eingeführt wird, um dann die Intraokularlinse mittels des Injektors über die Injektorspitze einzuführen. Insbesondere ist vorgesehen, dass eines der Kassettenteile positionsfixiert mit der Injektorspitze verbunden ist und somit keine Relativbewegung zwischen der Injektorspitze und diesem Kassettenteil ermöglicht ist. Das bzw. die dann noch verbleibende(n) weitere(n) Kassettenteil(e) ist/sind dann relativ zu der Injektorspitze und zu dem anderen Kassettenteil verschwenkbar.

Insbesondere ist vorgesehen, dass das relativ zu dem anderen Kassettenteil und zur Injektorspitze verschwenkbare Kassettenteil mit dem Einfüllloch ausgebildet ist.

Der Injektor weist vorzugsweise auch einen Kolben auf, der axial verschiebbar ist. Mittels dieses Kolbens kann dann eine axiale Ausschiebung der Intraokularlinse aus der Kassette in Richtung einer Injektorspitze des Injektors erfolgen. Insbesondere wird die in der Kassette durch den Schließvorgang der Kassette bereits vorgefaltete Intraokularlinse dann auf dem weiteren Ausschiebeweg in der Injektorspitze in ihre endgültige Faltlage gebracht und kann dann beim Austritt aus der Injektorspitze entsprechend gefaltet in das Auge eingeführt werden.

Mit Angaben "oben", "unten", "vorne", "hinten, "horizontal", "vertikal", "außen", "innen" etc. sind die bei bestimmungsgemäßen Gebrauch und bestimmungsgemäßem Anordnen der Kassette, insbesondere an einem Injektor zur Injektion einer Intraokularlinse in eine Auge, gegebenen Positionen und Orientierungen angegeben.

Ausführungsbeispiele der Erfindung werden nachfolgend anhand schematischer Zeichnungen näher erläutert. Es zeigen:
- Fig. 1: eine perspektivische Darstellung eines Ausfiihrungsbeispiels eines erfindungsgemäßen Injektors mit einem Ausführungsbeispiel einer erfindungsgemäßen Kassette;
- Fig. 2: eine perspektivische Darstellung eines ersten Ausführungsbeispiels einer erfindungsgemäßen Kassette im geöffneten Zustand;
- Fig. 3: eine vergrößerte Darstellung der Ansicht in Fig. 2;
- Fig. 4: eine perspektivische Darstellung der Kassette gemäß Fig. 2 und Fig. 3 im geschlossenen Zustand;
- Fig. 5: eine Darstellung gemäß Fig. 4 mit einem zusätzlichen Hilfswerkzeug zur Einführung eines Gleitmittels in die Kassette;
- Fig. 6: eine Querschnittsdarstellung senkrecht zur Längsachse der Kassette gemäß Fig. 2 bis Fig. 5 im geschlossenen Zustand der Kassette und mit einer in einer Aufnahmekammer der Kassette enthaltenen und vorgefalteten Intraokularlinse;
- Fig. 7: eine perspektivische Darstellung des ersten Ausführungsbeispiels der Kassette gemäß Fig. 2 bis 6;
- Fig. 8: eine vergrößerte Darstellung eines Teilausschnitts der Kassette gemäß Fig. 2 bis Fig. 7;
- Fig. 9: eine perspektivische Darstellung eines zweiten Ausführungsbeispiels einer erfindungsgemäßen Kassette;
- Fig. 10: eine Querschnittsansicht der Kassette gemäß der zweiten Ausführungsform im geschlossenen Zustand der Kassette;
- Fig. 11: eine perspektivische Darstellung eines dritten Ausführungsbeispiels einer erfindungsgemäßen Kassette im geöffneten Zustand der Kassette;
- Fig. 12: eine Querschnittsansicht des dritten Ausführungsbeispiels der erfindungsgemäßen Kassette im geschlossenen Zustand;
- Fig. 13: eine perspektivische Darstellung der Kassette gemäß Fig. 12 im geschlossenen Zustand;
- Fig. 14: eine perspektivische Darstellung eines vierten Ausführungsbeispiels einer erfindungsgemäßen Kassette im geöffneten Zustand;
- Fig. 15: eine perspektivische Darstellung der Kassette gemäß Fig. 14 im geschlossenen Zustand und mit einem zusätzlichen Hilfswerkzeug zum Einführen eines Gleitmittels in die Kassette;
- Fig. 16: eine Querschnittsansicht senkrecht zur Längsachse der Kassette gemäß Fig. 15;
- Fig. 17: eine vergrößerte Darstellung eines Teilbereichs der Kassette gemäß Fig. 14 bis Fig. 16 mit einer vergrößerten Darstellung eines Zuführkanals;
- Fig. 18: eine Querschnittsansicht eines fünften Ausführungsbeispiels einer erfindungsgemäßen Kassette im geschlossenen Zustand; und
- Fig. 19: eine perspektivische Darstellung der Kassette gemäß Fig. 18 im geöffneten Zustand.

In den Figuren werden gleiche oder funktionsgleiche Elemente mit den gleichen Bezugszeichen versehen.

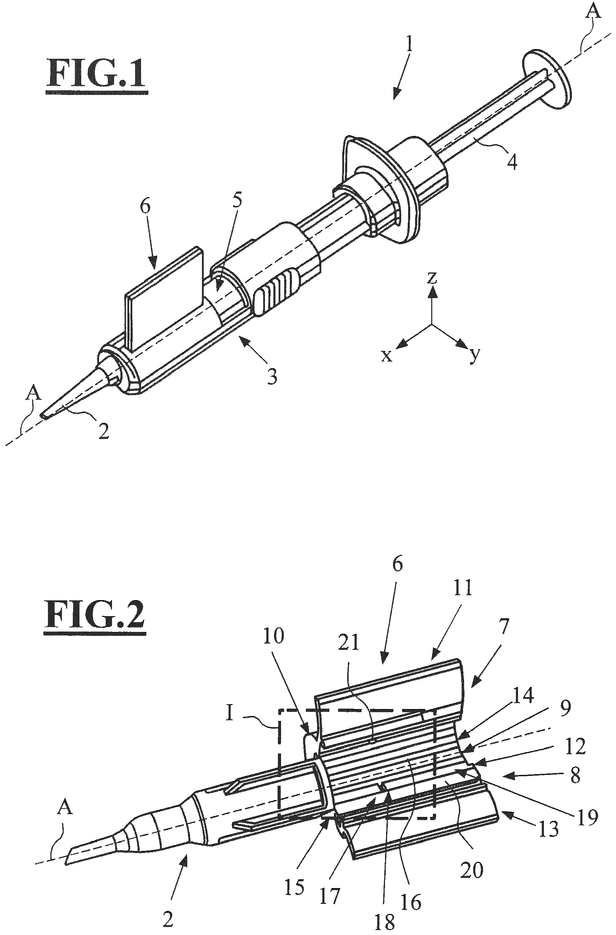

In Fig. 1 ist in einer perspektivischen Darstellung ein Injektor 1 gezeigt. Der Injektor 1 ist zum Einführen einer Intraokularlinse in ein Auge ausgebildet. Die lediglich schematisch zu verstehende Darstellung des Injektors 1 zeigt eine Injektorspitze 2, die in Richtung einer Längsachse A des Injektors 1 frontseitig angeordnet ist. Die Injektorspitze 2 wird dann bei einer Implantation bereichsweise in das Auge eingeführt und die Intraokularlinse über die Injektorspitze 2 ins Auge implantiert.

Der Injektor 1 umfasst darüber hinaus ein Injektorrohr 3, welches in axialer Richtung nach hinten an die Injektorspitze 2 anschließt. Der Injektor 1 umfasst darüber hinaus einen Kolben 4, der in Richtung der Längsachse A in dem Injektorrohr 3 verschiebbar ist. In dem Injektorrohr 3 ist auch ein Ladebereich 5 ausgebildet, in dem eine Kassette 6 einsetzbar ist oder darin fest positioniert ist.

Die zu implantierende Intraokularlinse ist in der Kassette 6 aufgenommen. Die Kassette 6 ist in axialer Richtung und somit in Richtung der Längsachse A frontseitig und rückseitig offen, sodass die sich darin befindliche Intraokularlinse durch den Kolben 4 in Richtung der Injektorspitze 2 aus der Kassette 6 ausgeschoben werden kann und in die Injektorspitze 2 eingeschoben werden kann.

Die Kassette 6 kann ein für sich betrachtet eigenes, separates Bauteil sein. Sie kann jedoch auch einstückig mit der Injektorspitze 2 ausgebildet sein.

In Fig. 2 ist in einer perspektivischen Darstellung ein erstes Ausführungsbeispiel der Kassette 6 gezeigt.

Generell ist die Kassette 6 bei allen Ausführungen als einstückiges Bauteil, insbesondere aus Kunststoff, ausgebildet.

Die Kassette 6 ist bei dem in Fig. 2 gezeigten Beispiel einstückig mit der Injektorspitze 2 ausgebildet. Sie kann jedoch auch separat dazu ausgebildet sein. Auch diese beiden Möglichkeiten sind für die dann auch noch nachfolgend erläuterten Ausführungsbeispiele möglich.

Die Kassette 6 umfasst ein Kassettenteil 7 sowie ein weiteres Kassettenteil 8. Die beiden Kassettenteile 7 und 8 sind um eine Längsachse der Kassette 6, die der Längsachse A entspricht, relativ zueinander verschwenkbar. Die Kassettenteile 7 und 8 sind bei dieser Ausführungsform direkt miteinander verbunden, insbesondere über ein Filmscharnier 9. Die Kassettenteile 7 und 8 können jedoch auch an einem oder mehreren Sockelelementen verbunden sein, wobei die Kassettenteile 7 und 8 um dieses Sockelelement oder diese Sockelelemente verschwenkbar sind.

Das Kassettenteil 7 umfasst ein rinnenartiges beziehungsweise bauchiges Basiselement 10 und einen daran anschließenden Flügel 11. Der Flügel 11 ist plattenartig ausgebildet.

Das Kassettenteil 8 umfasst ebenfalls ein rinnenartiges beziehungsweise bauchiges Basiselement 12 und einen daran anschließenden Flügel 13. Die Kassette 6 ist an einem axial hinteren Ende 14 ebenso offen ausgebildet, wie an einem axial vorderen Ende 15. Das axial vordere Ende 15 mündet direkt an die Injektorspitze 2.

In Fig. 2 ist die Kassette 6 im geöffneten Zustand gezeigt. Wird die Kassette 6 geschlossen, so werden die beiden Kassettenteile 7 und 8 um die Achse A aufeinander zu verschwenkt und die beiden Flügel 11 und 13 miteinander verbunden, insbesondere mittels einer Schnappverbindung.

Durch die entgegengesetzt gekrümmten rinnenartigen Basiselemente 10 und 12 wird im geschlossenen Zustand der Kassette 6 eine röhrenförmige Aufnahmekammer 16 für die Intraokularlinse gebildet.

Um die Intraokularlinse aus der Kassette 6 in die Injektorspitze 2 mit möglichst geringer Reibung aus dem Injektor auszuschieben, wird ein Gleitmittel, insbesondere ein viskoelastisches Medium, insbesondere ein OVD, in die Kassette eingeführt.

Dies erfolgt im geschlossenen Zustand der Kassette 6. Da es gerade im geschlossenen Zustand der Kassette 6 bezüglich der Zugänglichkeit schwierig ist, dieses Gleitmittel zuzuführen, ist diesbezüglich eine Gleitmittel-Zuführeinrichtung 17 vorgesehen, die derartiges entsprechend einfach ermöglicht. Durch die Gleitmittel-Zuführvorrichtung 17 ist es darüber hinaus im geschlossenen Zustand der Kassette auch erreicht, dass das in die Aufnahmekammer 16 zugeführte Gleitmittel möglichst gleichmäßig eingebracht und verteilt wird, sodass die darin befindliche Intraokularlinse nicht unerwünscht verschoben wird oder ein weiterer Faltvorgang der Linse nicht unerwünscht beeinträchtigt wird.

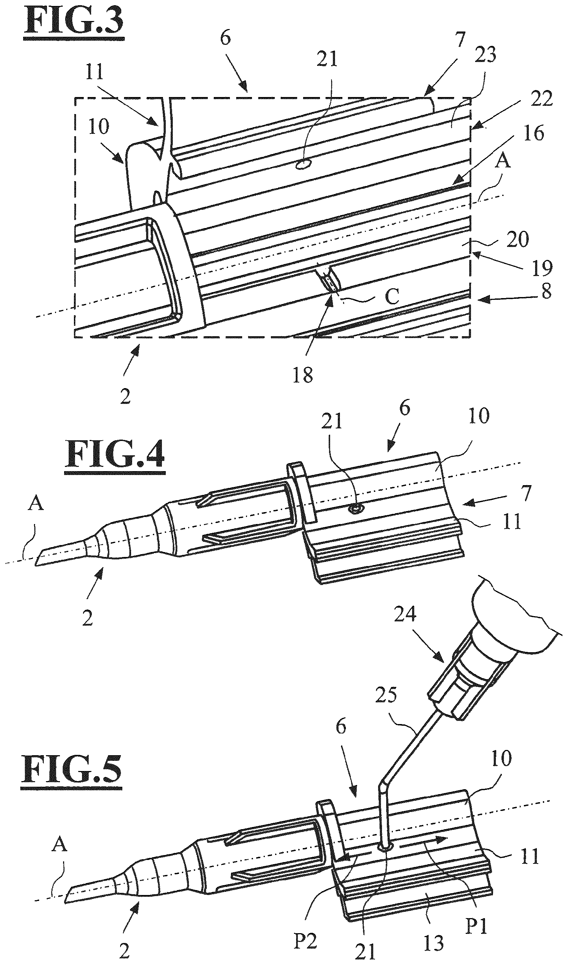

Dazu weist die Gleitmittel-Zuführeinrichtung 17 in der in Fig. 2 gezeigten Ausführung einen ersten Zuführkanal 18 auf, der in dieser Ausführungsform rinnenartig ausgebildet ist, vgl. auch Fig. 3. Dieser erste rinnenartige Zuführkanal 18 ist im Kassettenteil 8 ausgebildet. In der gezeigten Ausführung ist das Kassettenteil 8 dasjenige, welches positionsfixiert mit der Injektorspitze 2 verbunden ist. Demgegenüber ist das Kassettenteil 7 relativ zu der Injektorspitze 2 und zu dem Kassettenteil 8 verschwenkbar.

Der erste rinnenartige Zuführkanal 18, der durch die Rinnenform ein radial nicht vollständig geschlossener Kanal ist, verläuft entlang einer Längsachse C. Diese Längsachse C ist in der in Fig. 3 gezeigten Ausführungsform in einem Winkel zwischen 80° und 100°, insbesondere 90° gegenüber der Längsachse A orientiert. Dieser erste rinnenartige Zuführkanal 18 mündet somit radial in die Aufnahmekammer 16 und ist geradlinig ausgebildet.

An dem Kassettenteil 8 ist ein Steg 19 ausgebildet, der sich in die Aufnahmekammer 16 radial hineinerstreckt. Dieser Steg 19 ist im Ausführungsbeispiel parallel zur Längsachse A ausgebildet und insbesondere über die gesamte axiale Länge des Kassettenteils 8 gebildet. Der Steg 19 weist eine Oberseite 20 auf, die insbesondere eben ausgebildet ist. Diese Oberseite 20 bildet somit auch eine Innenseite, die im geschlossenen Zustand der Kassette 6 dem Kassettenteil 7 zugewandt ist. In dieser Oberseite 20 ist der erste rinnenartige Zuführkanal 18 ausgebildet und nach oben hin beziehungsweise aus der Oberseite 20 heraus offen gebildet. Er ist somit über seine gesamte Erstreckung und somit seine gesamte Länge dem Kassettenteil 7 zugewandt offen ausgebildet.

Der erste rinnenartige Zuführkanal 18 ist bei der in Fig. 3 dargestellten Ausführungsform vollständig geradlinig ausgebildet. Im geschlossenen Zustand der Kassette 6 mündet dieser erste rinnenartige Zuführkanal nicht nach außen, sodass er von außen nicht direkt zugänglich ist.

Es kann auch vorgesehen sein, dass der rinnenartige Zuführkanal 18 entlang seiner Kanalachse nichtgeradlinig ausgebildet ist. Der rinnenartige Zuführkanal 18 kann gemäß einer weiteren Ausführungsform einfach oder mehrfach gekrümmt und damit "schlangenförmig" ausgebildet sein oder mit einem Winkelversatz entlang seiner Kanalachse versehen sein. Eine derartige Ausgestaltung erhöht den Strömungswiderstand des Zuführkanals und kann bei einem Gleitmittel mit geringer Viskosität die Geschwindigkeit begrenzen, mit der das Gleitmittel in die Aufnahmekammer eindringt.

Die Gleitmittel-Zuführeinrichtung 17 umfasst darüber hinaus ein Einfüllloch 21, welches in dem Kassettenteil 7 ausgebildet ist, sodass das Einfüllloch 21 und der erste rinnenartige Zuführkanal 18 in unterschiedlichen Kassettenteilen ausgebildet sind.

Das Einfüllloch 21 ist als durchgehendes Loch ausgebildet und mündet im geschlossenen Zustand der Kassette 6 nach außen. Das Einfüllloch 21 ist so ausgebildet, dass im geschlossenen Zustand der Kassette 6 das Einfüllloch 21 in den ersten rinnenartigen Zuführkanal 18 radial mündet beziehungsweise mit diesem derart korrespondiert, dass ein über die Einfüllöffnung beziehungsweise das Einfüllloch 21 zugeführtes Gleitmittel in den ersten rinnenartigen Zuführkanal 18 gelangt und von dort in die Aufnahmekammer 16 eingeleitet wird.

In Fig. 3 ist eine vergrößerte Darstellung eines Ausschnitts I in Fig. 2 gezeigt. Das Einfüllloch 21 ist dabei in einem Steg 22 an dem Kassettenteil 7 ausgebildet. Der Steg 22 ist ebenfalls keilförmig ausgebildet und erstreckt sich in Richtung der Längsachse A über die gesamte Erstreckung des Kassettenteils 7. Der Steg 22 weist eine Oberseite 23 beziehungsweise eine Innenseite auf, die im geschlossenen Zustand der Kassette 6 an der Oberseite 20 des Stegs 19 anliegt. Der Steg 22 ist an einem innenseitigen Übergang zwischen dem Basiselement 10 und dem Flügel 11 ausgebildet. Entsprechend ist auch der Steg 19 an einem innenseitigen Übergang zwischen dem Basiselement 12 und dem Flügel 13 ausgebildet. Die Stege 19 und 22 bilden auch Faltanschlagelemente, sodass das Vorfalten der Intraokularlinse in der Kassette 6 selbst beim Schließen der Kassette 6 in azimutaler Richtung begrenzt ist.

Indem im geschlossenen Zustand der Kassette 6 die Oberseiten 20 und 23 flächig aufeinanderliegen, ist der erste rinnenartige Zuführkanal 18 in radialer Richtung auch über seine Länge geschlossen, insbesondere über diese Länge vollständig geschlossen.

In Fig. 4 ist die Ausgestaltung des Moduls mit der Injektorspitze 2 und der Kassette 6 im geschlossenen Zustand der Kassette 6 gezeigt. Eine Mündungsöffnung des Einfülllochs 21 ist zu erkennen. Es wird örtlich daher eine sehr spezifische Schnittstelle und Einmündungsstelle zum Einführen des Gleitmittels an der Kassette 6 geschaffen.

In besonderer Vorteilhaftigkeit ist es dadurch auch bei diesem Ausführungsbeispiel und bei allen weiteren Ausführungsbeispielen erreicht, dass die Mündungsöffnung des Einfüllloches 21 außerhalb des Basiselementes vorgesehen ist.

Der erste rinnenartige Zuführkanal 18 ist auf einer dem Filmscharnier 9 gegenüberliegenden Seite angeordnet. Es erfolgt somit kein Einleiten des Gleitmittels im Bereich des Filmscharniers 9 und nicht im Bereich von Enden des Basiselements 10, die den Flügeln 11 und 13 abgewandt sind. Durch diese Ausgestaltung wird auch erreicht, dass das Gleitmittel stets von der Seite in die Aufnahmekammer 16 eingeleitet wird, die der gewünschten Faltrichtung der Intraokularlinse hin zu den Basiselementen 10 und 12 nicht hindernd entgegensteht, sondern dies begünstigt. Es wird also der beim Schließen der Kassette 6 erreichte vorgefaltete Zustand der Intraokularlinse durch das Einführen des Gleitmittels nicht teilweise aufgehoben oder in die entgegengesetzte Richtung bewirkt. Stattdessen lässt sich durch die dann erzielte und im Querschnitt U-förmige Vorfaltung der Intraokularlinse das Gleitmittel in den durch die U-Form der vorgefalteten Intraokularlinse offenen Bereich einführen, sodass der vorgefaltete Zustand der Intraokularlinse durch das Gleitmittel sogar noch gestützt wird. Die Verteilung des Gleitmittels ist dann diesbezüglich auch noch entsprechend begünstigt, sodass ein gleichmäßiges Verteilen und gleichmäßiges Einbringen in die Aufnahmekammer 16 erreicht wird und somit dann ein verbessertes Ausschiebeverhalten der Intraokularlinse aus der Aufnahmekammer 16 bewirkt wird.

In Fig. 5 ist in einer perspektivischen Darstellung ein Hilfswerkzeug 24 gezeigt, welches mit einer Injektionsnadel 25 ausgebildet ist. Mittels dieses Hilfswerkzeugs 24 wird das darin befindliche Gleitmittel in die dazu externe Kassette 6 über die Einfüllöffnung 21 und den ersten rinnenartigen Zuführkanal 18 in die Aufnahmekammer 16 eingebracht. Durch die nur als Beispiel angegebenen Pfeile P1 und P2 ist die diesbezügliche gleichmäßige Verteilung des Gleitmittels angedeutet.

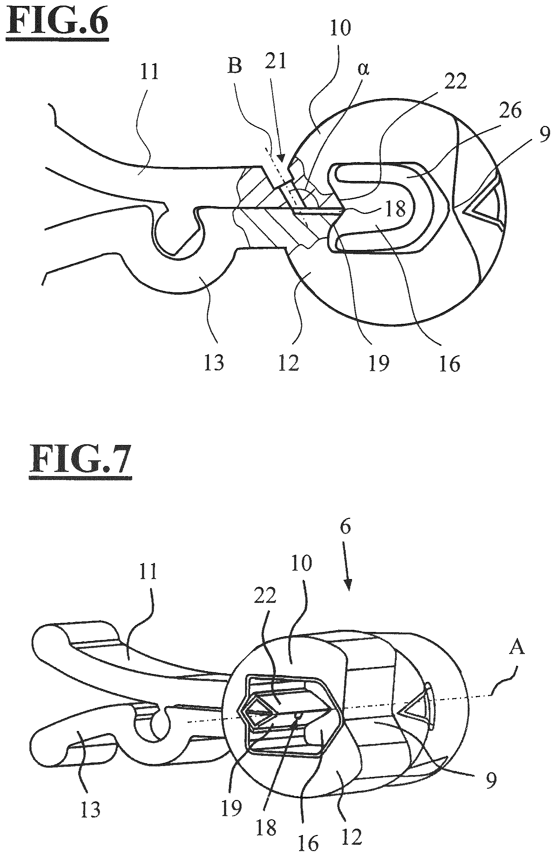

In Fig. 6 ist in einer schematischen Schnittdarstellung in einer Schnittebene senkrecht zur Längsachse A die Kassette 6 gemäß der ersten Ausführungsform im geschlossenen Zustand gezeigt. Wie zu erkennen ist, ist eine Lochachse B in einem Winkel α zu einer Längsachse C des ersten rinnenartigen Zuführkanals 18 orientiert. Der Winkel α beträgt vorzugsweise zwischen 90° und 140°.

Darüber hinaus ist in Fig. 6 auch die Erstreckung der Stege 19 und 22 in Richtung zur Aufnahmekammer 16 zu erkennen, die zur Horizontalen geneigte Anschlagflächen für die Intraokularlinse aufweisen. Diese Anschlagflächen bilden ein Anschlagelement, welches als azimutaler Anschlagstopp für die Intraokularlinse 26 dient, die in Fig. 6 in einem U-förmig vorgefalteten Zustand in der geschlossenen Aufnahmekammer 16 gezeigt ist. Es ist auch zu erkennen, dass durch diesen vorgefalteten Zustand die Intraokularlinse 26 zum Filmscharnier 9 hin gebogen ist und sich zum Steg 19 und zum Steg 22 hin die Öffnung der U-Form ergibt. Durch diese Lage und die Ausgestaltung der Gleitmittel-zuführeinrichtung 17 kann dann in diesem Freiraum der gefalteten Intraokularlinse 26 gezielt das Gleitmittel eingebracht und umfänglich in der Aufnahmekammer 16 verteilt werden.

Bei dieser ersten Ausführungsform ist der rinnenartige Zuführkanal 18 ausschließlich in dem Steg 19 vorgesehen. Im Steg 22 ist ausschließlich die Einfüllöffnung 21 vorgesehen. Im geschlossenen Zustand der Kassette 6 ist es somit möglich, das Gleitmittel von der Einfüllöffnung 21 zu dem rinnenartigen Zuführkanal 18 und von dort in die Aufnahmekammer 16 zu befördern.

In Fig. 7 ist in einer perspektivischen Darstellung die Kassette 6 gemäß der ersten Ausführungsform gezeigt. Insbesondere sind die Stege 19 und 22 mit ihrer schrägen Anschlagfläche gut erkennbar. Auch ist die Einmündung des ersten rinnenartigen Zuführkanals 18 in die Aufnahmekammer 16 gezeigt.

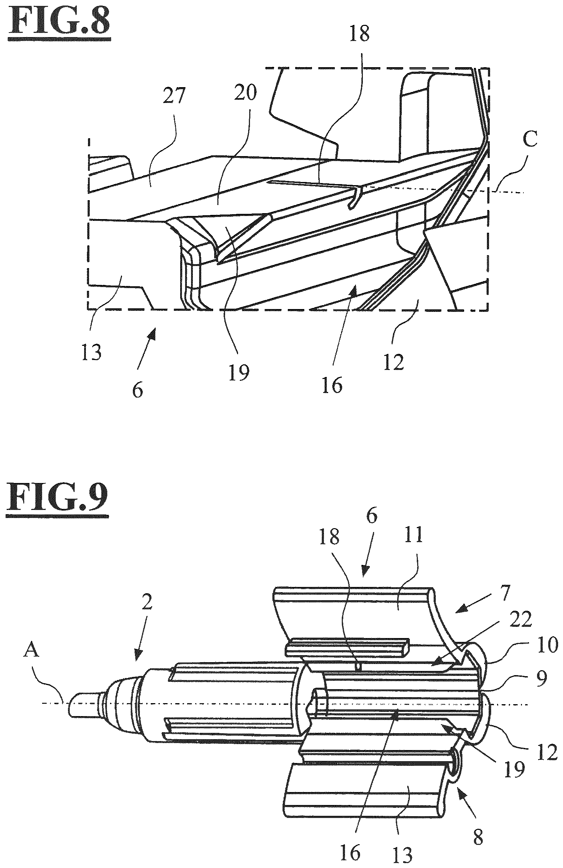

In Fig. 8 ist in einer vergrößerten Darstellung ein Ausschnitt der Kassette 6 im Bereich des Stegs 19 mit dem ersten rinnenförmigen Zuführkanal 18 gezeigt, wobei die Kassette 6 im geöffneten Zustand ist. Es ist zu erkennen, dass die Oberseite 20 bündig in eine Oberseite beziehungsweise Innenseite 27 des Flügels 13 übergeht. Wie darüber hinaus auch zu erkennen ist, erstreckt sich der erste rinnenartige Zuführkanal 18 mit seiner Längsachse C senkrecht zur Längserstreckung des Steges 19. Der Zuführkanal 18 kann jedoch auch unterhalb der Oberseite 20 verlaufen und ist dann nicht mehr rinnenförmig, sondern entlang seines gesamten Umfangs geschlossen ausgebildet. Der Zuführkanal bildet dann ein Sackloch, welches bei geschlossenem Zustand der Kassette 6 mit dem Einfüllloch 21 korrespondiert.

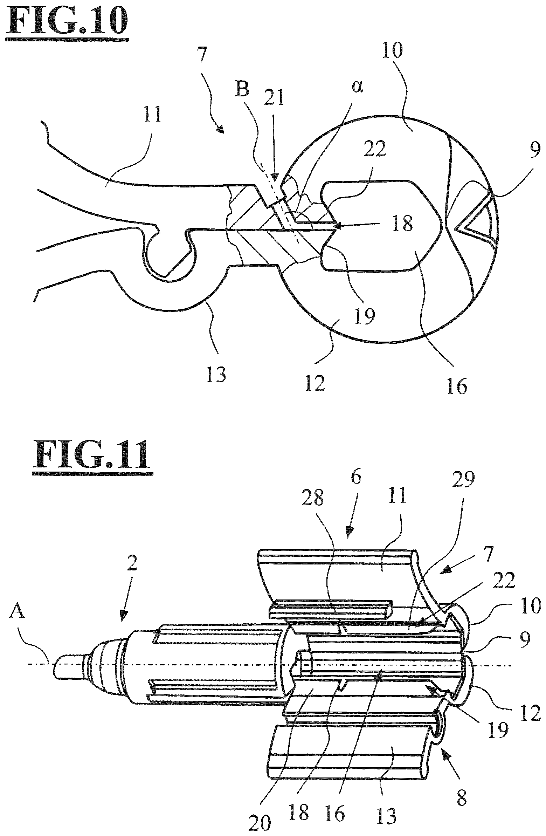

In Fig. 9 ist in einer perspektivischen Darstellung und in Fig. 10 in einem Querschnitt ein zweites Ausführungsbeispiel einer Kassette 6 im geöffneten Zustand gezeigt. Auch hier ist als Beispiel eine einstückige Ausgestaltung der Kassette 6 mit der Injektorspitze 2 gezeigt, wobei diese auch anderweitig ausgestaltet sein kann und die Kassette 6 als separates Teil ausgebildet sein kann. Im Unterschied zu dem ersten Ausführungsbeispiel ist hier vorgesehen, dass sowohl der erste rinnenartige Zuführkanal 18 als auch das Einfüllloch 21 in einem gemeinsamen Kassettenteil, insbesondere in dem Kassettenteil 7, ausgebildet sind. Die Lochachse B des Einfülllochs 21 ist in einem Winkel α von bevorzugt 90° bis 140° zur Längsachse C des rinnenartigen Zuführkanals 18 ausgebildet. Das Kassettenteil 7 ist das schwenkbare Kassettenteil.

In Fig. 11 ist in einer perspektivischen Darstellung ein drittes Ausführungsbeispiel einer Kassette 6 im geöffneten Zustand gezeigt. Als Beispiel ist hier die Kassette 6 wiederum einstückig mit der Injektorspitze 2 verbunden. Bei dieser Ausgestaltung ist vorgesehen, dass ein erster rinnenartiger Zuführkanal 18 im Kassettenteil 8 und somit in dem feststehenden Kassettenteil 8 ausgebildet ist. Insbesondere ist er im Steg 19 ausgebildet, wie dies bereits bei den vorhergehenden Ausführungsbeispielen erläutert wurde.

Darüber hinaus ist das Einfüllloch 21 in dem Kassettenteil 7 ausgebildet. Zusätzlich ist hier vorgesehen, dass in diesem Kassettenteil 7 auch ein zweiter rinnenartiger Zuführkanal 28 ausgebildet ist. Die Gleitmittel-Zuführeinrichtung 17 umfasst somit den rinnenartigen Zuführkanal 18 und den rinnenartigen Zuführkanal 28. Das Einfüllloch 21 mündet direkt in den zweiten rinnenartigen Zuführkanal 28. Dieser zweite rinnenartige Zuführkanal 28 ist insbesondere in dem Steg 22 ausgebildet und zu der Oberseite 23 hin offen ausgebildet. Im geschlossenen Zustand der Kassette 6 ist hier vorgesehen, dass die Oberseiten 23 und 20 wie bei der ersten Ausführungsform flächig aufeinander liegen und die rinnenartigen Zuführkanäle 18 und 28 so ausgebildet sind, dass sie im geschlossenen Zustand der Kassette 6 deckungsgleich aufeinander liegen. Sie decken sich daher gegenseitig ab. Es wird dadurch ein im Querschnitt und somit im Volumen größerer Gesamtzuführkanal aus den beiden rinnenartigen Zuführkanälen 18 und 28 gebildet. Auch der zweite rinnenartige Zuführkanal 28 erstreckt sich über eine Länge, die der Breite des Stegs 22 entspricht und ist somit als Sackkanal ausgebildet, der nur mit einem offenen Ende in axialer Richtung des zweiten rinnenartigen Zuführkanals 28 betrachtet in die Aufnahmekammer 16 mündet.

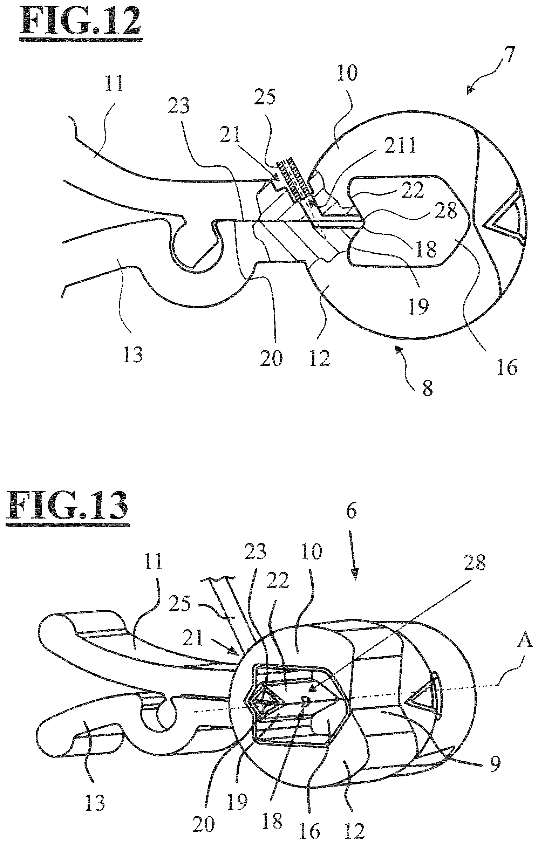

In Fig. 12 ist die dritte Ausführungsform der Kassette 6 im geschlossenen Zustand in einem Querschnitt gezeigt, wobei keine Intraokularlinse im Aufnahmeraum 16 dargestellt ist. Ferner ist in Fig. 12 die Injektionsnadel 25 des Hilfswerkzeuges 24 gezeigt. Es ist dabei zu erkennen, dass auch bei einer entsprechenden Einstechtiefe der Injektionsnadel 25 in das Einfüllloch 21 niemals ein kompletter Durchstich bis zur Aufnahmekammer 16 auftreten kann. Die Injektionsnadel 25 kann maximal bis zu einem Boden 211 des Einfüllloches 21 vorgeschoben werden. Dies ist vorteilhaft, da somit kein Berührkontakt mit einer eventuellen Beschädigung einer Intraokularlinse 26 im Aufnahmeraum 16 möglich ist.

In Fig. 13 ist in einer perspektivischen Darstellung die dritte Ausführungsform einschließlich der Injektionsnadel 25 gezeigt, wobei hier die Kassette 6 im geschlossenen Zustand dargestellt ist. Das direkte Aufeinanderliegen der Oberseiten 20 und 23 ist hier gezeigt.

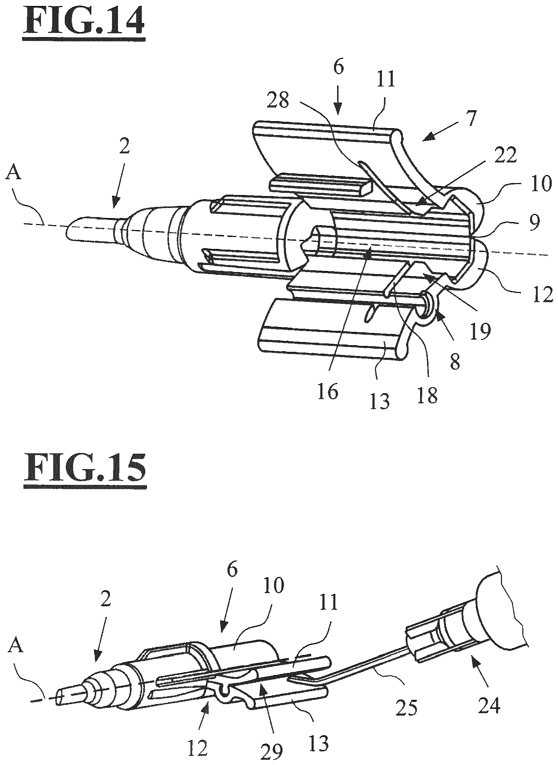

In Fig. 14 ist in einer perspektivischen Darstellung ein viertes Ausführungsbeispiel einer Kassette 6 gezeigt. Auch hier ist im Beispiel eine einstückige Ausgestaltung mit der Injektorspitze 2 vorgesehen.

Bei dieser Ausgestaltung ist die Gleitmittel-Zuführeinrichtung 17 mit einem ersten rinnenartigen Zuführkanal 18 ausgebildet, der in dem Kassettenteil 8 gestaltet ist. Ein zweiter rinnenartiger Zuführkanal 28 ist in dem Kassettenteil 7 gebildet. Beide rinnenartigen Zuführkanäle 18 und 28 sind vollständig geradlinig ausgebildet.

Der erste rinnenförmige Zuführkanal 18 erstreckt sich entlang einer Längsache C, welche in einem Winkel zwischen 80° und 100°, insbesondere 90°, gegenüber der Längsachse A orientiert ist. Der erste rinnenartige Zuführkanal 18 erstreckt sich von der Aufnahmekammer 16 beginnend in eine Richtung zu einem freien äußeren Ende des Flügels 13. Die Länge des ersten rinnenartigen Zuführkanals 18 ist so gewählt, dass im geschlossenen Zustand der Kassette 6 dieser rinnenartige Zuführkanal 18 direkt von außen zugänglich ist. Entsprechendes gilt auch für den zweiten rinnenartigen Zuführkanal 28.

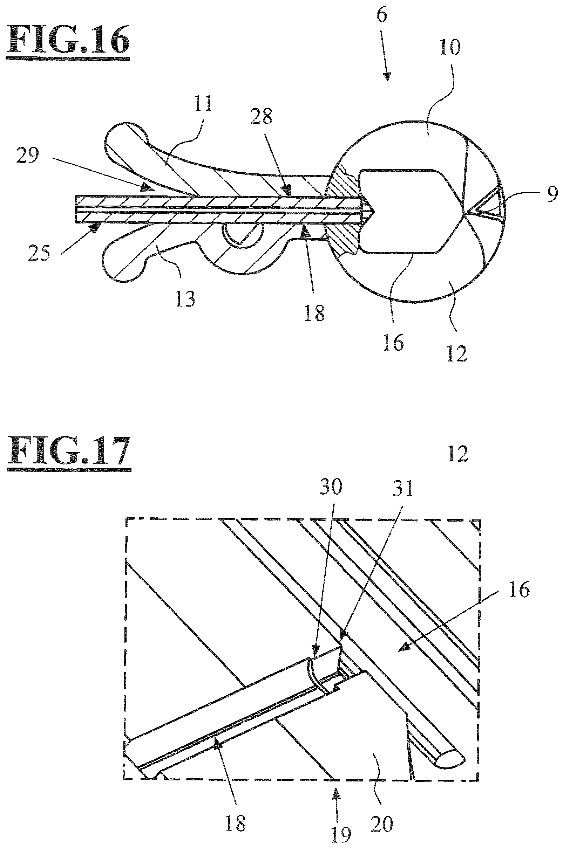

Bei dieser Ausführung ist es somit erreicht, dass, wie dies in Fig. 15 und Fig. 16 dargestellt ist, im geschlossenen Zustand der Kassette 6 die Injektionsnadel 25 an einer Eingangsstelle 29 direkt in den Gesamtzuführkanal, der aus den beiden rinnenartigen Zuführkanälen 18 und 28 gebildet ist, eingeführt werden kann.

Wie darüber hinaus in Fig. 14 und 15 zu erkennen ist, sind die Flügel 11, 13 an ihren den Basiselementen 10 und 12 abgewandten freien Enden voneinander weg gekrümmt, sodass eine schnabelartige Aufweitung entsteht, in die die Injektionsspitze 25 eingeführt werden kann. Durch diese Ausgestaltung endet der Gesamtzuführkanal vor den freien Enden der Flügel 11 und 13. Dadurch ist er auch im gewissen Maße geschützt und das Einführen der Injektionsnadel 25 ist entsprechend zielführend möglich und dadurch auch entsprechend geführt.

In Fig. 16 ist in einer schematischen Querschnittsdarstellung senkrecht zur Längsachse A die Ausgestaltung der Kassette 6 gemäß Fig. 14 und 15 gezeigt.

Um insbesondere bei dieser Ausführung, jedoch kann dies auch bei anderen Ausführungen möglich sein, den Eintritt der Injektionsnadel 25 in die Aufnahmekammer 16 zu vermeiden, ist zumindest ein rinnenartiger Zuführkanal 18 und/oder 28 an seinem in die Aufnahmekammer 16 mündenden Ende 30 mit einer Verjüngung 31 ausgebildet, siehe Fig. 17. Insbesondere können beide rinnenartigen Zuführkanäle 18 und 28 an ihren der Aufnahmekammer 16 zugewandten und in die Aufnahmekammer 16 mündenden Enden verjüngt ausgebildet sein. Die Verjüngung ist dabei derart, dass eine Durchmesserreduzierung erreicht wird, sodass die Injektionsnadel 25 beim Einführen in den Gesamtzuführkanal an der Verjüngung 31 gestoppt wird und nicht in die Aufnahmekammer 16 eintreten kann. Die Verjüngung 31 kann als diskrete Stufe oder als stetige, beispielsweise konusförmige Verjüngung ausgebildet sein.

Darüber hinaus kann es bei einer weiteren Ausführung auch vorgesehen sein, dass sich die Stege 19 und 22 in radialer Richtung und somit hin zur Längsachse A mit unterschiedlichen Abmessungen ausbilden und somit ein der Aufnahmekammer 16 zugewandter Rand beziehungsweise ein freies Ende eines Stegs 19 oder 22 sich weiter in die Aufnahmekammer 16 hineinerstreckt als dies bei dem anderen Steg der Fall ist. Bei einem derartigen radialen Überstand in die Aufnahmekammer 16 hinein kann die Funktionalität als Faltanschlag nochmals individualisiert werden. Insbesondere ist dann vorgesehen, dass der erste rinnenartige Zuführkanal 18 in dem Steg ausgebildet ist, der sich weiter in die Aufnahmekammer 16 hineinerstreckt als der andere Steg. Dadurch wird nicht nur eine axiale Öffnung dieses rinnenartigen Zuführkanals erzielt, sondern darüber hinaus auch noch eine radiale Öffnung erzielt, die durch den Überstand dieses Stegs im Vergleich zu dem anderen Steg erreicht ist.

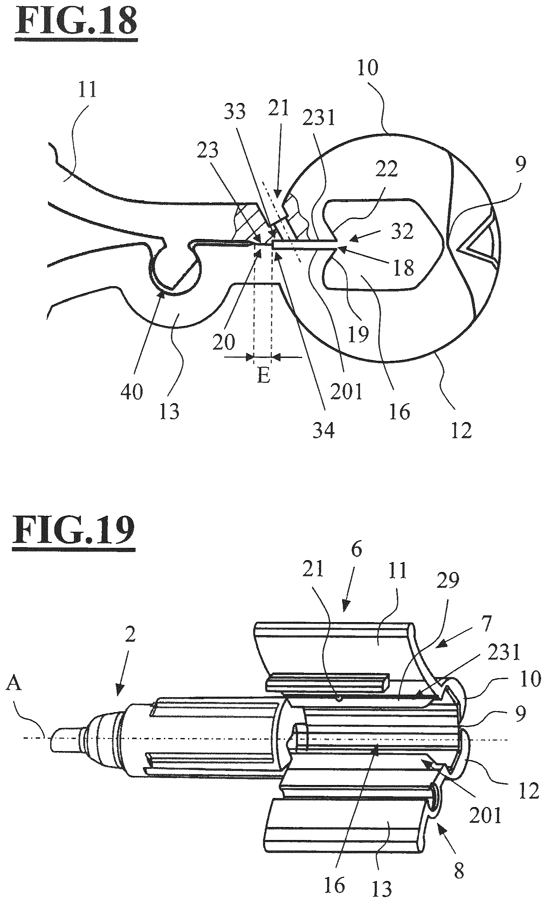

In Fig. 18 ist eine fünfte Ausführungsform gemäß der Erfindung dargestellt. Der Flügel 11 ist mit einer Einfüllöffnung 21 versehen, welche geeignet ist, die Injektionsnadel 25 aufzunehmen. Die Einfüllöffnung 21 mündet an einer Oberfläche 231 des Steges 22, welche gegenüber der Oberfläche 23 des Steges in Richtung zum Aufnahmeraum 16 abgesetzt ist. Zwischen der Oberfläche 23 und der Oberfläche 231 besteht somit eine abfallende Stufe 33, die sich entlang der gesamten Länge des Steges 22 erstreckt. Im geschlossenen Zustand der Kassette 6 berührt die Oberfläche 23 die Oberfläche 20 des Flügels 13.

Bevorzugt ist die Oberfläche 20 in Richtung zum Aufnahmeraum 16 ebenfalls abgesetzt, wodurch eine Oberfläche 201 gebildet ist, die sich entlang der gesamten Länge des Steges 19 erstreckt. Zwischen der Oberfläche 201 und der Oberfläche 20 ist somit eine abfallende Stufe 34 gebildet. Die Kontaktebene zwischen der Oberfläche 20 und der Oberfläche 23 bildet bevorzugt eine Symmetrieebene bezüglich der abgesetzten Oberflächen 201 und 231, sodass die jeweiligen Höhen der Stufen 33 und 34 den gleichen Betrag einnehmen.

Der Bereich zwischen der abgesetzten Oberfläche 231 und der Oberfläche 20 oder zwischen der abgesetzten Oberfläche 231 und der abgesetzten Oberfläche 201 ergibt einen Freiraum 32, der einen Zuführkanal 18 bzw. eine Zuführöffnung für das Gleitmittel entlang der gesamten Länge der Stege 19 und 22 bildet. Das Gleitmittel kann damit sehr breit verteilt in den Aufnahmeraum 16 eindringen. Damit ist es möglich, relativ viel Gleitmittel sehr schnell in den Aufnahmeraum 16 zu verteilen.

Die Flächen 20 und 23 müssen sich im geschlossenen Zustand der Kassette 6 derart berühren, dass ein flüssigkeitsdichter Abschluss gegenüber dem mittels der Einfüllöffnung 21 eingebrachten Gleitmittel sichergestellt ist. Bei der in Fig. 18 dargestellten Ausführungsform berühren sich die Flächen 20 und 23 entlang einer Breite E. Es ist jedoch auch möglich, dass die Breite größer ausgeführt ist und bis zur Schnappverbindung 40 der Flügel 11 und 13 reicht.

In Fig. 19 ist in einer perspektivischen Darstellung die Ausgestaltung der Kassette 6 gemäß Fig. 18 im geöffneten Zustand dargestellt.

## Patentansprüche

1. Kassette (6) zur Aufnahme einer Intraokularlinse (26), mit zwei um eine Längsachse (A) der Kassette (6) relativ zueinander schwenkbaren Kassettenteilen (7, 8), wobei ein Kassettenteil (7) ein rinnenartiges Basiselement (10) und einen daran anschließenden plattenartigen Flügel (11) aufweist, und ein weiteres Kassettenteil (8) ein rinnenartiges Basiselement (12) und einen daran anschließenden plattenartigen Flügel (13) aufweist, und im geschlossenen Zustand der Kassette (6) durch die beiden Basiselemente (10, 12) mindestens ein Teil einer Aufnahmekammer (16) für die Intraokularlinse (26) gebildet ist, und mit einer Gleitmittel-Zuführeinrichtung (17), mit welcher bei geschlossener Kassette (6) ein Gleitmittel für die Intraokularlinse (26) von außerhalb der Kassette (6) in die Aufnahmekammer (16) zuführbar ist, wobei die Gleitmittel-Zuführeinrichtung (17) einen ersten Zuführkanal (18) aufweist, der in einem Kassettenteil (7, 8) ausgebildet ist und in die Aufnahmekammer (16) mündet,
**dadurch gekennzeichnet, dass**
der erste Zuführkanal (18) in einem sich in die Aufnahmekammer (16) hinein erstreckenden Steg (19) eines Kassettenteils (7) ausgebildet ist.

2. Kassette (6) nach Anspruch 1,
**dadurch gekennzeichnet, dass**
der erste Zuführkanal (18) geradlinig ausgebildet ist.

3. Kassette (6) nach Anspruch 1 oder 2,
**dadurch gekennzeichnet, dass**
sich der Steg (19) parallel zur Längsachse (A) erstreckt und in Richtung der Längsachse (A) betrachtet sich zumindest über die Hälfte der Länge der Aufnahmekammer (16) geradlinig erstreckt.

4. Kassette (6) nach einem der Ansprüche 1 bis 3,
**dadurch gekennzeichnet, dass**
der an einem Kassettenteil (7) ausgebildete Steg (19) eine dem anderen Kassettenteil (8) zugewandte Oberseite (20) aufweist, in welcher der erste Zuführkanal (18) rinnenartig ausgebildet und zur Oberseite (20) hin offen ausgebildet ist.

5. Kassette (6) nach einem der Ansprüche 1 bis 4,
**dadurch gekennzeichnet, dass**
der Steg (19) keilartig ausgebildet ist und/oder an einem innenseitigen Übergang zwischen einem Basiselement (10, 12) und einem Flügel (11, 13) eines Kassettenteils (7, 8) ausgebildet ist.

6. Kassette (6) nach einem der Ansprüche 1 bis 5,
**dadurch gekennzeichnet, dass**
in dem anderen Kassettenteil (8) ein sich in die Aufnahmekammer (16) erstreckender weiterer Steg (22) ausgebildet ist, in dem ein zweiter Zuführkanal (28) ausgebildet ist, und im geschlossenen Zustand der Kassette (6) die beiden Zuführkanäle (18, 28) deckungsgleich angeordnet sind und sich gegenseitig abdecken.

7. Kassette (6) nach einem der vorhergehenden Ansprüche,
**dadurch gekennzeichnet, dass**
der erste Zuführkanal (18) in einem Kassettenteil (7) ausgebildet ist und ein durchgängiges Einfüllloch (21) an dem oder dem weiteren Kassettenteil (7, 8) ausgebildet ist, wobei das Einfüllloch (21) im geschlossenen Zustand der Kassette (6) an dem ersten Zuführkanal (18) mündet und im geschlossenen Zustand der Kassette (6) nach außen mündet.

8. Kassette (6) nach einem der vorhergehenden Ansprüche,
**dadurch gekennzeichnet, dass**
zumindest der erste Zuführkanal (18) in einem Winkel zwischen 60° und 120° zu der Längsachse (A) der Kassette (6) orientiert ist.

9. Kassette (6) nach einem der Ansprüche 1 bis 8,
**dadurch gekennzeichnet, dass**
der zumindest erste Zuführkanal (18) mit einem der Aufnahmekammer (16) abgewandten Ende im geschlossenen Zustand der Kassette (6) an dem Flügel (11, 13) eines Kassettenteils (7, 8) nach außen hin mündet und der erste Zuführkanal (18) geradlinig ausgebildet ist.

10. Injektor (1) zum Einführen einer Intraokularlinse (26) in ein Auge, welcher eine Kassette (6) nach einem der vorherigen Ansprüche aufweist.

## Claims

1. Cartridge (6) for receiving an intraocular lens (26), having two cartridge parts (7, 8) that are pivotable relative to one another about a longitudinal axis (A) of the cartridge (6), wherein one cartridge part (7) has a channel-like base element (10) and a plate-like wing (11) adjoining the latter, and a further cartridge part (8) has a channel-like base element (12) and a plate-like wing (13) adjoining the latter, and in the closed state of the cartridge (6), at least a part of a receiving chamber (16) for the intraocular lens (26) is formed by the two base elements (10, 12), and having a lubricant feed device (17) with which, with the cartridge (6) closed, a lubricant for the intraocular lens (26) is feedable into the receiving chamber (16) from outside the cartridge (6), wherein the lubricant feed device (17) has a first feed duct (18), which is formed in one cartridge part (7, 8) and leads into the receiving chamber (16),
**characterized in that**
the first feed duct (18) is formed in a rib (19), extending into the receiving chamber (16), of one cartridge part (7).

2. Cartridge (6) according to Claim 1,
**characterized in that**
the first feed duct (18) is formed in a rectilinear manner.

3. Cartridge (6) according to Claim 1 or 2,
**characterized in that**
the rib (19) extends parallel to the longitudinal axis (A) and, as seen in the direction of the longitudinal axis (A), extends in a rectilinear manner at least along half the length of the receiving chamber (16).

4. Cartridge (6) according to one of Claims 1 to 3,
**characterized in that**
the rib (19) formed on one cartridge part (7) has a top side (20) facing the other cartridge part (8), the first feed duct (18) being formed in a channel-like manner in said top side (20) and being formed in an open manner toward the top side (20).

5. Cartridge (6) according to one of Claims 1 to 4,
**characterized in that**
the rib (19) is formed in a wedge-like manner and/or is formed at an internal transition between a base element (10, 12) and a wing (11, 13) of one cartridge part (7, 8) .

6. Cartridge (6) according to one of Claims 1 to 5,
**characterized in that**
a further rib (22) that extends into the receiving chamber (16) is formed in the other cartridge part (8), a second feed duct (28) being formed in said further rib (22), and in the closed state of the cartridge (6), the two feed ducts (18, 28) are arranged in a congruent manner and cover one another.

7. Cartridge (6) according to one of the preceding claims,
**characterized in that**
the first feed duct (18) is formed in one cartridge part (7) and a continuous filling hole (21) is formed in the or the further cartridge part (7, 8), wherein the filling hole (21) leads into the first feed duct (18) in the closed state of the cartridge (6) and leads toward the outside in the closed state of the cartridge (6).

8. Cartridge (6) according to one of the preceding claims,
**characterized in that**
at least the first feed duct (18) is oriented at an angle of between 60° and 120° to the longitudinal axis (A) of the cartridge (6).

9. Cartridge (6) according to one of Claims 1 to 8,
**characterized in that**
the at least first feed duct (18) leads, with an end facing away from the receiving chamber (16), toward the outside at the wing (11, 13) of one cartridge part (7, 8) in the closed state of the cartridge (6), and the first feed duct (18) is formed in a rectilinear manner.

10. Injector (1) for introducing an intraocular lens (26) into an eye, said injector (1) having a cartridge (6) according to one of the preceding claims.

## Revendications

1. Cassette (6) servant à la réception d'une lentille intraoculaire (26), comportant deux parties de cassette (7, 8) pouvant pivoter l'une par rapport à l'autre autour d'un axe longitudinal (A) de la cassette (6), une partie de cassette (7) comprenant un élément de base (10) en forme de rigole et une ailette (11) en forme de plaque s'y raccordant, et une autre partie de cassette (8) comprenant un élément de base (12) en forme de rigole et une ailette (13) en forme de plaque s'y raccordant, et à l'état fermé de la cassette (6), au moins une partie d'une chambre de réception (16) pour la lentille intraoculaire (26) étant formée par les deux éléments de base (10, 12), et comportant un dispositif d'alimentation en lubrifiant (17), à l'aide duquel un lubrifiant pour la lentille intraoculaire (26) peut être acheminé depuis l'extérieur de la cassette (6) dans la chambre de réception (16) lorsque la cassette (6) est fermée, le dispositif d'alimentation en lubrifiant (17) comprenant un premier canal d'alimentation (18) qui est réalisé dans une partie de cassette (7, 8) et débouche dans la chambre de réception (16),
**caractérisée en ce que**
le premier canal d'alimentation (18) est réalisé dans une nervure (19), s'étendant dans la chambre de réception (16), d'une partie de cassette (7).

2. Cassette (6) selon la revendication 1,
**caractérisée en ce que**
le premier canal d'alimentation (18) est réalisé de manière rectiligne.

3. Cassette (6) selon la revendication 1 ou 2,
**caractérisée en ce que**
la nervure (19) s'étend parallèlement à l'axe longitudinal (A) et, considérée dans la direction de l'axe longitudinal (A), s'étend de manière rectiligne au moins sur la moitié de la longueur de la chambre de réception (16).

4. Cassette (6) selon l'une des revendications 1 à 3,
**caractérisée en ce que**
la nervure (19) réalisée sur une partie de cassette (7) comprend un côté supérieur (20) tourné vers l'autre partie de cassette (8), dans lequel le premier canal d'alimentation (18) est réalisé en forme de rigole et est réalisé de manière ouverte vers le côté supérieur (20).

5. Cassette (6) selon l'une des revendications 1 à 4,
**caractérisée en ce que**
la nervure (19) est réalisée en forme de coin et/ou est réalisée au niveau d'une transition intérieure entre un élément de base (10, 12) et une ailette (11, 13) d'une partie de cassette (7, 8).

6. Cassette (6) selon l'une des revendications 1 à 5,
**caractérisée en ce**
**qu'**une autre nervure (22) s'étendant dans la chambre de réception (16) est réalisée dans l'autre partie de cassette (8), nervure dans laquelle un deuxième canal d'alimentation (28) est réalisé, et les deux canaux d'alimentation (18, 28) sont disposés en coïncidence et se recouvrent mutuellement à l'état fermé de la cassette (6) .

7. Cassette (6) selon l'une des revendications précédentes,
**caractérisée en ce que**
le premier canal d'alimentation (18) est réalisé dans une partie de cassette (7) et un trou de remplissage traversant (21) est réalisé sur la ou l'autre partie de cassette (7, 8), le trou de remplissage (21) débouchant au niveau du premier canal d'alimentation (18) à l'état fermé de la cassette (6) et débouchant vers l'extérieur à l'état fermé de la cassette (6).

8. Cassette (6) selon l'une des revendications précédentes,
**caractérisée en ce**
**qu'**au moins le premier canal d'alimentation (18) est orienté suivant un angle compris entre 60° et 120° par rapport à l'axe longitudinal (A) de la cassette (6).

9. Cassette (6) selon l'une des revendications 1 à 8,
**caractérisée en ce que**
l'au moins un premier canal d'alimentation (18) débouche vers l'extérieur au niveau de l'ailette (11, 13) d'une partie de cassette (7, 8) par une extrémité opposée à la chambre de réception (16) à l'état fermé de la cassette (6) et le premier canal d'alimentation (18) est réalisé de manière rectiligne.

10. Injecteur (1) servant à l'introduction d'une lentille intraoculaire (26) dans un œil, lequel injecteur comprend une cassette (6) selon l'une des revendications précédentes.
